# EUROPEAN PATENT SPECIFICATION

(11) **EP 0 238 005 B1**
(45) Date of publication and mention of the grant of the patent: **20.07.1994**
(21) Application number: 87103754.5
(22) Date of filing: 16.03.1987
(51) Int. Cl.: A61M 5/31, A61M 5/34, A61M 5/24

(54) **High viscosity fluid delivery system**
System zur Abgabe von hochviskosen Flüssigkeiten
Système pour délivrer des fluides à haute viscosité

(30) Priority: 20.03.1986 US 841971
(43) Date of publication of application: 23.09.1987
(73) Proprietor: Orentreich, Norman, New York New York 10021 (US)
(72) Inventor: Orentreich, Norman, New York, New York 10021 (US); Vogelman, Joseph Herbert, Roslyn, New York 11576 (US)
(74) Representative: Koepsell, Helmut, Dipl.-Ing.

(56) References cited:
- US-A- 2 537 550
- US-A- 3 368 557
- US-A- 3 392 726
- US-A- 3 537 453
- US-A- 3 730 389
- US-A- 3 848 593
- US-A- 3 958 570

## Description

The invention relates to a carpule system for the microadministration of fluid including a carpule syringe according to the first part of claim 1.

Carpule syringes adapted for the microadministration of highly viscous liquids such as silicone fluid, are known in the art. U.S. patent 3,958,570 discloses syringe capsules which are factory sealed and contain the liquid to be administered, the delivery needle being integral with the capsule as part of the unit assembled in the factory.

However, this prior publication is silent on the design and arrangement of such combination of needle and capsule and on the concrete arrangement for holding and securing the capsule in the syringe.

The present invention is concerned with the problem of providing a carpule syringe for the microadministration of high viscosity fluid comprising a delivery needle secured to one end of the carpule which is easy to handle especially with regard to holding and securing the carpule in the syringe. Another aspect concerns the design of connecting means for connecting delivery needle and carpule.

The present invention provides a remedy to the a.m. problem by application of the features in the characterizing part of claim 1.

Claim 2 pertains to details as to the means for connecting the delivery needle to the carpule.

For the purpose of illustrating the invention there is shown in the drawings an enbodiment which is presently preferred, it being understood, however, that the invention is not limited to the precise arrangements and instrumentalities shown.
Fig. 1 is an exploded partially sectioned side elevational view of a syringe in accordance with the present invention;
Fig. 2 is a fragmentary end-view of the housing of the syringe of Fig. 1;
Fig. 3 is an enlarged sectional view of the carpule and portion of the syringe of Fig. 1;
Fig. 4 is an enlarged sectional view of the needle portion of the syringe of Fig. 1.

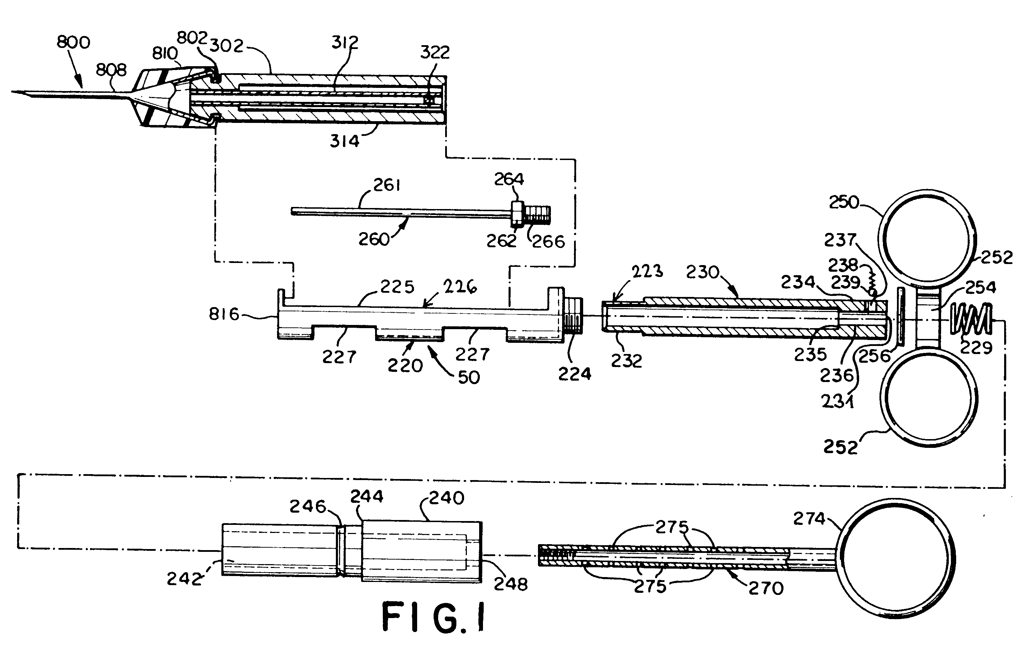

Referring now to the drawings, in which like reference numerals identify like elements, there is shown in Fig. 1 an exploded partially sectioned side elevational view of a preferred embodiment of a syringe 50 according to the present invention.

In the application, the term "proximal", when used in reference to an element of a syringe or carpule, refers to the forward end (left end when viewing Fig. 1) thereof which is proximate the delivery needle. Similarly, the term "distal" when used here in reference to an element of the syringe or carpule refers to the rearward end (right end when viewing Fig. 1) thereof which is situated away from the delivery needle. Unless otherwise indicated, all of the components of the syringe of the present invention are preferably made of stainless steel or some other material having similar properties.

The size of the needle chosen for use with syringes of the present invention depends on the dermatological application for which the syringe is used. For example, when silicone fluid is to be inserted behind a fine line in the skin, a needle having a small diameter, such as 30 gauge needle, is preferred for accuracy. A relatively large hydraulic force is required to make the relatively viscous silicone fluid flow through the bore of a needle having a small diameter. The necessary force is obtained by use of a syringe having a bore with a cross-sectional area which is substantially greater than the corresponding area of the delivery needle. In human dermatological applications it has been found that delivery needles having an interior diameter from about 1 mm (0.004 inches) to 1.8 mm (0.007 inches) are most frequently useful. Other applications may require needles having different interior diameters.

In order to apply sufficient force on the viscous fluid, which for dermatological application typically is a silicone fluid having a viscosity ranging from about 150 to 350 centistokes, it is preferred that the bore of the syringe have a cross-sectional area about 100 to 400 times greater than the cross-sectional area of the delivery needle. Syringes which are intended for use with fine needles and viscous silicone fluids for human dermatological use typically have a capacity in the order of 0.5 ml.

In the preferred embodiment, illustrated in Fig. 1 to 4, the present invention provides a delivery system adapted for injecting high viscosity fluid contained within prepackaged carpules which are inserted into the syringe.

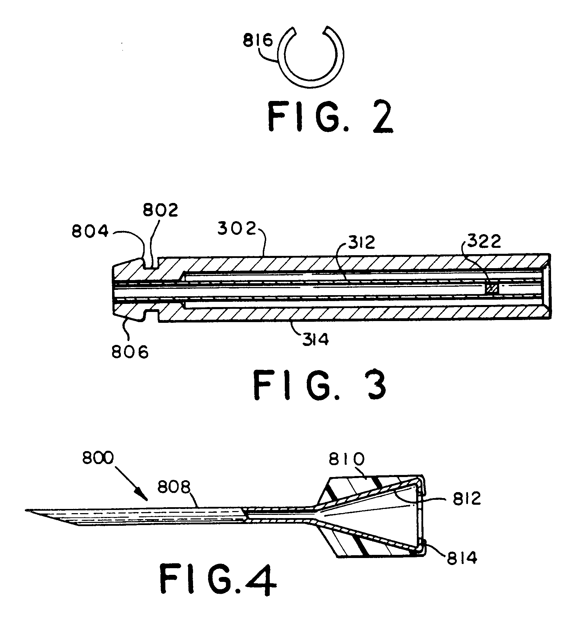

The carpule 302 illustrated in Fig. 3 includes an interior tube or wall 312 preferably manufactured from stainless steel tubing or a material having similar properties which contains the fluid to be delivered. The exterior housing 314 of the carpule 302 is preferably made of aluminium or a similar material. The carpule 302 is sealed at one end by a polytetrafluorethylene plug 322. The delivery needle 800 is secured directly to the carpule 302 in a manner which will hereinafter be described in greater detail.

Referring now to Fig. 3 and 4, there is shown, in greater detail, an enlarged sectional view of the carpule 302 and needle 800 portions of the syringe 50 of Fig. 1. The forward or proximal end of the carpule 302 includes a generally annular groove 802 extending around the radial circumference. For purposes which will hereinafter become apparent, the edge 804 of the groove 802 proximate the forward end of the carpule 302 is beveled or rounded. The portion 806 of the carpule 302 extending forward (toward the left one viewing Fig. 3) from the groove 802 is beveled or inwardly tapered, the taper being preferably a luer lock taper.

Correspondingly, the needle 800 is of a type well known in the art and is comprised of a special extra thick flanged generally tubular delivery portion 808 which is supported within a generally cylindrical connector member 810. The rearward end of the connector member 810 includes an interior beveled or tapered portion 812 which is sized and tapered to compliment the tapered portion 806 on the forward end of the carpule 302. Thus, when the needle 800 and the carpule are joined together as shown in Fig. 1, the tapered portions 806 and 812 compliment each other to provide a fluid type seal between the needle 800 and the carpule 302.

The rearward end of the connector member 810 further includes an annual flange member 814 (see Figs. 1 and 4). When the needle 800 is installed on the carpule 302 the annular flange member 814 is formed during assembly when the end of the needle connector member 810 is bent or peened inwardly and into the groove 802 as shown in Fig. 1. For clarity, Fig. 4 shows the annular flange member 814 in the position it assumes after installation on the carpule 302. The combination of the engagement of the two tapered portions 806 and 812 and the engagement of the flange member 814 with the groove 802 firmly secures the needle 800 to the forward end of the carpule 302.

The generally cylindrical housing 220 of the carpule syringe 50 has a large elongated medial opening 225 of sufficient length and depth so that a carpule may be inserted through the opening 225 into the assembled syringe 50. Small medial openings 227 in the housing 220 facilitate insertion and removal of carpules. The distal end 224 of housing 220 has exterior threads which are adapted to engage interior threads on interior bore 242 of a cap 240.

The carpule syringe 50 is provided with means to urge and maintain carpule 302 into abutting engagement with the proximal end of the housing 220. The means comprises a coil carpule spring 229, preferably manufactured from spring steel, and having an exterior diameter less than the diameter of the bore of cap 240. The carpule spring 239 exerts sufficient force when compressed as described below so that carpule 302 is held tightly within the housing 220.

A generally cylindrical adapter member or adapter 230 having a first or large axial bore 231 of sufficiently large inside diameter to permit a plunger rod assembly 260 to pass therethrough is provided. The proximal end 232 of the adapter 230 has a reduced outer diameter and a beveled end 233 to engage and retain the carpule 302 within the housing 220 when the carpule syringe 50 is assembled. Proximate the distal end 234 of the adapter 230, the adapter 230 has a second or small axial with a bore 236 reduced cross-sectional area. A shoulder 235 is formed between the sections of the bore having different interior diameters. As discussed below, the shoulder 235 serves to limit the withdrawal of the plunger rod assembly 260 from the syringe when the syringe is loaded with a carpule.

The distal end 234 of the adapter 230 includes detent means, in the present embodiment, a detent hole 237 extending generally perpendicular to the axis and a detent ball 239 partially protruding into the bore 231 of the adapter 230. A detent spring 238 is provided to urge the detent ball 239 towards the bore 231.

The interior bore 242 of cap 240 is greater in diameter than the largest exterior diameter of adapter 230 and which is adapted to receive the carpule spring 229 and the adapter 230. The proximal end of interior bore 242 is threaded to engage the exterior threads on the distal end 224 of housing 220 to hold the adapter 230 and cap 240 in place. The exterior surface of cap 240 is provided with a generally annular shoulder 244 and a generally annular groove 246.

A finger 250 is provided which comprises finger loops 252 connected to a generally cylindrical grip mount 254. The interior radius of the grip mount 254 is substantially the same as the radius of the exterior surface of the cylindrical cap 240 such that the grip mount 254 is adapted to receive the exterior surface of the cylindrical cap 240. A generally annular spring clip 256, preferably manufactured from spring steel, is adapted to be received in annular groove 246. The spring clip 256 is adapted to press the grip mount 254 against the annular shoulder 244 to hold the finger grip 250 on cap 240. Other finger grip geometries may be used. The finger grip 250 and cap 240 may be preassembled.

The syringe plunger includes plunger rod assembly 260 and a plunger extension 270. The plunger rod assembly 260 comprises a plunger rod 261, having a diameter slightly less than the interior diameter of the carpule bore, and a plunger adapter 262. The distal end of the plunger rod 260 is threaded to engage interior threads on the proximal end of the plunger adapter 262 when the syringe is assembled. The plunger adapter 262 includes a hexagonal head 264 having a diameter less then the diameter of the large diameter bore 231 of the adapter 230, but greater than the diameter of the small diameter bore 236 of the adapter 230.

The generally tubular plunger extension 270 may be inserted through the hole in wall 248 into the cap 240. The distal end 266 of the plunger adapter 262 has exterior threads which are adapted to engage threads on the proximal end of the plunger extension 270. The head 264 of the plunger rod assembly 260 serves to prevent the plunger from being withdrawn from the syringe when the carpule 302 is loaded into the syringe. The plunger extension 270 is terminated at its distal end by a thumb grip means or thumb ring 274. Instead of the thumb ring 274, parts having other geometries adapted to firmly engage the thumb of the syringe user may be employed.

Spaced, generally annular detent grooves 275 are located on the exterior surface of the plunger extension 270, preferably at a separation of 0.14 cm (0.05 inches). The grooves 275 are sequentially engaged by the spring loaded detent ball 239 as the plunger is moved inwardly through the carpule syringe 50. This action tactilely signals advance of the plunger to the individual administering fluid using the syringe.

For assembling carpule syringe 50 the finger grip 250 is placed in position on the cap 240 against the annular shoulder 244 and the spring clip 256 is inserted into the annular groove 246 to hold the finger grip 250 in position. Alternatively, the finger grip 250 may be attached to the cap 240 after the cap 240 is threaded onto housing 220. Preferably, the finger grip 250, spring clip 256, and cap 240 form a subassembly.

The carpule spring 229 is inserted into the distal end 224 of the cap 240. The detent ball 239 is then inserted into the detent hole 237 of the adapter 230 so that the detent ball partially protrudes into the central bore of the adapter 230. The detent spring 238 is inserted into the detent hole 237 and is compressed while the adapter 230 is inserted into the interior bore 242 of the cap 240. The cap 240 containing the adapter 230 is threaded onto the distal end 224 of the housing 220.

The plunger is assembled by screwing together the plunger rod assembly 260 within the syringe 50. The plunger extension 270 is first inserted through the hole in the wall 248 of the cap 240. The plunger extension 270 is positioned in the syringe 50 so that it protrudes within the housing 220. The plunger assembly 260 is then inserted through the large medial opening 225 in the housing 220 and secured to the end of the plunger extension 270.

Next, the plunger is withdrawn until the outward travel of the plunger is stopped by the abutment of the hexagonal head 264 of the plunger adapter 262 against the shoulder 235 within the bore of the adapter 230. Further withdrawal of the plunger compresses the carpule spring 229 positioned within the cap 240 between the adapter 230 and the end of the cap 240, permitting the carpule 302 to enter the housing 220 through the large medial opening 225.

It can be seen that the proximal or forward end of the cylindrical housing 220 includes a generally arcuate member 816 which comprises the forwardmost end of the housing 220. The arcuate member 816 is adapted to receive and engage the groove 802 on the forward end of the carpule 302 when the carpule is installed within the housing 220. The outer circumference of the arcuate member 816 is the same or slightly greater than the outer diameter of the carpule 302 while the inner diameter of the arcuate member 816 is the same or slightly greater than the diameter of the portion of the carpule surrounded by the groove 802. The carpule 302 is installed within the housing by aligning the carpule 302 with the housing opening 225 and pushing the carpule 302 and housing together. When the groove 802 initially engages the arcuate member 816 the unattached ends of the arcuate member 816 are spread apart to afford entry of the groove 802 and then spring back toward each other to hold the carpule 302 securely in place within the housing 220. The axial length of the groove 802 is sufficient to accommodate both the annular flange member 814 and the arcuate member 816.

Release of the plunger causes carpule spring 229 to urge the adapter 230 against carpule 302, thus securing the carpule 302 within syringe 50.

Removal of the carpule 302 from the housing 320 is accomplished by reversing the assembly process.

Plug 322 cooperates with plunger member 260 (Fig. 1) to move along the carpule tube 312 for delivering the fluid within the carpule 302.

## Claims

1. A carpule system for the microadministration of fluid including a carpule syringe (50) comprising
a generally fluid containing carpule (302) and a movable plug (322) sealing the fluid within the carpule (302);
a generally cylindrical housing member (220) having openings at each end and adapted to receive the carpule (302), the housing member (220) having means on one end for retaining the one end of the carpule;
a plunger (260) adapted to axially displace the carpule plug (322) within the carpule bore for delivery of the fluid;
the syringe (50) being for the microadministration of high viscosity fluid and comprising a delivery needle (800) secured to one end of the carpule (302), the needle having an interior diameter in the range of from about 0.11 mm (0.004 inches) to about 0.18 mm (0.007 inches) and the carpule (302) having a bore with a cross-sectional area which is about 100 to 400 times greater than the cross-sectional area of the bore of the delivery needle (800), one end of the carpule (302) including a tapered portion (806), and the needle (800) including a connector (810),
characterized in that
the carpule system comprises an adapter member (230) secured to the other end of the housing member (220) to secure the other end of the carpule (302) within the housing member (220) and adapter retention means (240) for securing the adapter member (230) to the other end of the housing member (220) and that the delivery needle (800) is made of stainless steel, wherein the connector (810) has a complementary tapered portion (812) to the tapered portion (806) of the carpule (302), the tapered portion (806) of the carpule (302) being adapted to sealingly engage the tapered portion (812) on the needle connector (810) for securing the needle (800) to the carpule (302) and providing a fluid tight seal.

2. A carpule system according to claim 1 wherein the carpule (302) further comprises a generally annular groove (802) extending around the circumference proximate the one end and wherein the needle connector (810) further includes a generally annular flange member (814), the flange member of the needle connector (810) being adapted to engage the carpule groove (802) to secure the needle (800) to the carpule (302).

## Patentansprüche

1. Carpulen-System mit einer Carpulen - Spritze (50) zum Mikro-Applizieren einer Flüssigkeit mit
einer allgemein eine Flüssigkeit enthaltenden Carpule (302) und einem bewegbaren Stopfen (322), der die Flüssigkeit innerhalb der Carpule einschließt;
einem allgemein zylindrischen Gehäuse (220), welches an jedem Ende mit Öffnungen versehen und so ausgebildet ist, daß es die Carpule (302) aufnehmen kann, wobei das Gehäuse (220) an einem Ende mit Mitteln versehen ist, um das eine Ende der Carpule zu halten;
einem Kolben (260), der in der Lage ist, den Carpulenstopfen (322) innerhalb der Carpulenbohrung zur Abgabe der Flüssigkeit axial zu verschieben,
wobei die Spritze (50) für das Mikro-Applizieren von hochviskosen Flüssigkeiten vorgesehen ist und eine Appliziernadel (800) aufweist, die an einem Ende der Carpule (302) befestigt ist und einen Innendurchmesser im Bereich von etwa 0.11 mm (0.004 inches) bis etwa 0.18 mm (0.007 inches) hat und die Carpule (302) eine Bohrung mit einem Querschnitt aufweist, der etwa 100 - 400mal größer ist als der Querschnitt der Bohrung der Appliziernadel (800), und ein Ende der Carpule (302) einen sich verjüngenden Abschnitt (306) aufweist und die Nadel (800) mit einem Verbindungsteil (810) versehen ist,
dadurch gekennzeichnet, daß
das Carpulen-System ein Zwischenstück (230) aufweist, welches am anderen Ende des Gehäuses (220) befestigt ist, um das andere Ende der Carpule (302) innerhalb des Gehäuses (220) zu halten, und Haltemittel (240) für das Zwischenstück vorgesehen sind, um das Zwischenstück (230) am anderen Ende des Gehäuses (220) zu halten und die Appliziernadel (800) aus rostfreiem Stahl hergestellt ist und das Verbindungsteil (810) einen zum sich verjüngenden Abschnitt (806) der Carpule (302) sich komplimentär verjüngenden Abschnitt (812) aufweist, und der sich verjüngende Abschnitt (806) der Carpule (302) so ausgeführt ist, daß er mit dem sich verjüngenden Abschnitt (812) am Nadel-Verbindungsteil (810) dichtend in Eingriff kommt, um die Nadel (800) an der Carpule (302) zu befestigen und einen flüssigkeitsdichtenden Verschluß zu bewirken.

2. Carpulen-System nach Anspruch 1 bei welchem die Carpule (302) mit einer allgemein ringförmigen Nut (802) versehen ist, die sich um den Umfang in der Nähe des einen Endes erstreckt, und das Nadel-Verbindungstteil (810) weiterhin ein allgemein ringförmiges Flanschteil (814) aufweist und das Flanschteil des Nadel-Verbindungsteils (810) so ausgebildet ist, daß es mit der Nut an der Carpule (802) in Eingriff bringbar ist, um die Nadel (800) an der Carpule (302) zu befestigen.

## Revendications

1. Système à "cartouche-ampoule", dite "carpule", pour administrer des microdoses de liquide au moyen d'une seringue (50) à carpule comportant une carpule (302) contenant un produit sensiblement liquide et pourvue d'un bouchon mobile (322) qui maintient le liquide enfermé de manière étanche à l'intérieur de la carpule (302); un corps (220) sensiblement cylindrique ouvert à ses deux extrémités et adapté à recevoir la carpule (302), ce corps (220) ayant à l'une de ses extrémités des moyens pour retenir l'une des extrémités de la carpule; un plongeur (260) prévu pour commander un déplacement axial du bouchon (322) de la carpule à l'intérieur de celle-ci, pour assurer le débit du liquide, la seringue (50) étant prévue pour administrer des microdoses d'un liquide de haute viscosité, et comportant une aiguille d'injection (800) fixée à une extrémité de la carpule (302), cette aiguille ayant un calibre interne compris entre environ 0,11 mm (0,004 pouce) et environ 0,18 mm (0,007 pouce), et la carpule ayant un alésage interne dont la section transversale présente une surface environ 100 à 400 fois plus grande que la surface de la section interne de l'aiguille d'injection (800), la carpule (302) ayant à l'une de ses extrémités une partie sensiblement tronconique (806), et l'aiguille (800) étant pourvue d'un raccord de fixation (810), caractérisé en ce que le système à carpule comporte un adaptateur (230) qui se fixe à l'autre extrémité du corps (220) pour fixer l'autre extrémité de la carpule (302) à l'intérieur du corps (220), et des moyens de retenue (240) associés à l'adaptateur (230) pour fixer celui-ci à l'autre extrémité du corps (220), et en ce que l'aiguille d'injection (800) est en acier inoxydable, le raccord de fixation (810) de celle-ci ayant une partie de profil sensiblement tronconique (812) complémentaire du profil tronconique de la première partie (806) de la carpule (302), cette partie tronconique (806) de la carpule (302) étant adaptée à se monter de manière étanche dans la partie tronconique (812) du raccord de fixation (810) de l'aiguille (800), pour fixer celle-ci sur la carpule (302) de manière étanche à l'égard des liquides.

2. Système à carpule selon la revendication 1, caractérisé en ce que la carpule (302) est pourvue en outre d'une gorge périphérique sensiblement annulaire (802) ménagée à proximité de la première extrémité de la carpule, et en ce que le raccord de fixation (810) de l'aiguille comporte en outre une lèvre en saillie sensiblement annulaire (814) adaptée à s'engager dans la gorge (802) de la carpule (302) pour fixer l'aiguille (800) sur la carpule (302).
